# EUROPÄISCHE PATENTANMELDUNG

(11) **EP 2 711 050 A1**
(43) Veröffentlichungstag der Anmeldung: **26.03.2014**
(21) Anmeldenummer: 12006607.1
(22) Anmeldetag: 20.09.2012
(51) Int. Cl.: A61Q 19/00, A61K 9/00, A61K 31/60

(54) **Zusammensetzung sowie kosmetische Zubereitung mit einer derartigen Zusammensetzung**

(71) Anmelder: Apotheker Walter Bouhon GmbH, 90427 Nürnberg (DE)
(72) Erfinder: Neufang, Hartmut, 91056 Erlangen (DE)
(74) Vertreter: FDST Patentanwälte

(57) **Zusammenfassung**

Es wird eine Zusammensetzung angegeben, umfassend 0,5 - 2,0 Gew.-% Salicylsäure oder nach INCI-Deklaration Salicylic Acid oder ihre anorganischen Salze, 0,5 - 5,0 Gew.-% Nicotinamid oder nach INCI-Deklaration Niacinamide, 0,05 - 0,5 Gew.-% Phytosphingosin oder nach INCI-Deklaration Phytosphingosine. 0 - 5,0 Gew.-% eines Kondensationsproduktes aus Azelainsäure oder nach INCI-Deklaration Azelaic Acid und Glycin oder nach INCI-Deklaration Glyzine oder seine Salze, sowie einen Rest. Eine derartige Zusammensetzung eignet sich insbesondere zur unterstützenden Pflege von unreiner Haut, insbesondere bei Jugendlichen. Weiter wird eine kosmetische und/oder dermatologische Zubereitung mit einer entsprechenden Zusammensetzung angegeben.

## Beschreibung

Die Erfindung betrifft eine Zusammensetzung zur unterstützenden Pflege von unreiner Haut, insbesondere zur unterstützenden Pflege von unreiner Haut bel Jugendlichen. Weiter betrifft die Erfindung eine entsprechende kosmetische und/oder dermatologische Zubereitung mit einer entsprechenden Zusammensetzung.

Unreine Haut und insbesondere Akne ist eine der am häufigsten auftretenden Hautkrankheiten. Bedingt durch hormonelle Veränderungen während der Pubertät treten Hautunreinheiten oder Akne hauptsächlich bei Jugendlichen auf. Mehr als 80 % der Jugendlichen und jungen Erwachsenen zwischen 10 und 25 Jahren sind davon betroffen. Die entzündlichen Hautveränderungen treten hierbei hauptsächlich im Gesicht auf. Aber auch Erwachsene können an derartigen Hautunreinheiten lelden.

Unreine Haut entsteht durch eine erhöhte Talgsekretion mit Verhornungsstörung der Talgdrüsenausführungsgänge. Die durch die Verhornung der Talgdrüsen entstehenden Mitesser und Pickel verhindern, dass der verstärkt gebildete Talg aus den Talgdrüsen abfließen kann, wodurch es zu Entzündungen kommen kann. Um diese Entzündungen zu bekämpfen und/oder ihnen zumindest vorzubeugen, ist insbesondere eine konsequente Reinigung und Pflege der betroffenen Hautpartien notwendig. Um Fettrückstände und Schmutz, beispielsweise von kosmetischen Produkten, zu entfernen, werden üblicherweise desinfizierende, zum Teil antibakterielle und mitesserlösende Substanzen in Gel-, Tinktur- oder Cremeform auf die Haut aufgebracht.

Derartige Produkte beinhalten herkömmlicherweise sowohl Inhaltsstoffe, die der Entstehung von Mitessern und Pickeln vorbeugen sollen, als auch solche, die die Heilung von Hautunreinheiten und Entzündungen beschleunigen. Gängige Inhaltsstoffe sind beispielsweise Salicylsäure und Niacinamide, deren entzündungshemmende und sebumreduzierende Wirksamkeit ausreichend erforscht und bestätigt ist.

Allerdings muss sowohl bei der Wahl der eingesetzten Inhaltsstoffe als auch bei der Einstellung der jeweiligen Konzentrationen berücksichtigt werden, dass bestimmte Inhaltsstoffe trotz ihrer bestätigten Wirksamkeit Unverlrägllchkeiten hervorrufen können. So kann es beispielsweise vorkommen, dass ein angewandtes Produkt zwar die Hautunreinheiten wirksam bekämpft, allerdings durch eine Unverträglichkeit eine Schädigung der Haut hervorgerufen wird. Aus diesem Grund richtet sich ein Augenmerk der Forschung auf die stetige Verbesserung von Zusammensetzungen und deren Anwendung in Hautpflegeprodukten.

Aufgabe der Erfindung ist es demnach, eine Zusammensetzung zur unterstützenden Pflege von unreiner Haut, insbesondere von Jugendlichen, anzugeben. Eine weitere Aufgabe der Erfindung ist es, eine kosmetische und/oder dermatologische Zubereitung mit einer entsprechenden Zusammensetzung anzugeben.

Die erstgenannte Aufgabe wird erfindungsgemäß durch eine kosmetische und/oder dermatologische Zusammensetzung gelöst, die 0,5 - 2,0 Gew.-% Salicylsäure oder nach INCI-Deklaration Salicylic Acid oder ihre anorganischen Salze, 0,5 - 5,0 Gew.-% Nicotinamid oder nach INCI-Deklaration Niacinamide, 0,05 - 0,5 Gew.-% Phytosphingosin oder nach INCI-Deklaration Phytosphingosine, 0 - 5,0 Gew.-% eines Kondensationsproduktes aus Azelainsäure oder nach INCI-Deklaration Azelaic Acid und Glycin oder nach INCI-Deklaration Glyzine oder seine Salze, sowie einen Rest umfasst.

Bei der Angabe der Inhaltsstoffe werden vorliegend sowohl die Namen der chemischen Verbindungen als auch die Namen gemäß INCI-Deklaration aufgeführt, da beide Bezeichnungen einander nicht immer entsprechen. Die INCI-Deklaration (International Nomenclature Cosmetic Ingredients: Internationale Nomenklatur für kosmetische Inhaltsstoffe) bezeichnet die internationale Angaberichtlinie von Inhaltsstoffen von Kosmetlka und erlaubt eine eindeutige Identifikation kosmetischer Inhaltsstoffe. Die Angabe der kosmetischen Inhaltsstoffe nach dem INCI-System ist in der EU seit 1997 gesetzlich vorgeschrieben und durch entsprechende Ländergesetze umgesetzt.

Die Erfindung beruht auf der Erkenntnis, dass zur Pflege von unreiner Haut die Kombination der jeweils eingesetzten Inhaltsstoffe berücksichtigt werden muss, um die gewünschte Wirksamkeit zu erreichen. Die Inhaltsstoffe bzw. die entsprechenden Zusammensetzungen sollen sowohl bereits bestehende Unreinheiten und Entzündungen bekämpfen als auch diesen vorbeugen.

Die Auswahl der Inhaltsstoffe und deren jeweilige Konzentrationen sollten hierbei optimalerweise so abgestimmt sein, dass sich die Wirkung der einzelnen Komponenten weder aufhebt noch unerwünscht verstärkt. Weiterhin ist eine Anwendbarkeit für unterschiedliche Hauttypen wünschenswert, wobei insbesondere allergische Reaktionen und Unverträglichkeiten verhindert werden sollen.

Durch eine Zusammensetzung, die 0,5 - 2,0 Gew.-% Salicylsäure oder nach INCI-Deklaration Salicylic Acid oder ihre anorganischen Salze, 0,5 - 5.0 Gew.-% Nicotinamid oder nach INGI-Deklaration Niacinamide, 0,05 - 0,5 Gew.-% Phytosphingosin oder nach INCI-Dekleratlon Phytsphingosine, 0,0-5,0 Gew.-% eines Kondensationsproduktes aus Azelainsäure oder nach INCI-Deklaration Azelaic Acid und Glycin oder nach INCI-Deklaration Glyzlne oder seine Salze, sowie einen Rest umfasst, können diese Anforderungen erfüllt werden.

Die Kombination der Inhaltsstoffe der Zusammensetzung in den angegebenen Konzentratlonsbereichen ermöglicht deren Anwendung insbesondere in Hautpflegeprodukten, besonders zur Pflege von unreiner Haut. Die Zusammensetzung kann zum Beispiel bei der Herstellung einer kosmetischen und/oder dermatologischen Zubereitung als O-W- oder als W-O-Emulsion eingesetzt werden. Insbesondere eignet sich die Zusammensetzung zur Herstellung einer Hautcreme, eines Reinigungsgels und/oder eines Gesichtswassers.

Da die eingesetzten Inhaltsstoffe der Zusammensetzung sich bezüglich ihrer Wirkung auf die Haut teilweise ergänzen, bzw. sich deren Wirkung verstärkt, ermöglicht eine derartige Zusammensetzung Insbesondere eine Variation der Konzentrationen der Inhaltsstoffe. So kann zum Beispiel die Konzentration von Salicylsäure erhöht werden, während die des Phylosphingosins verringert wird, da beide Substanzen keratolytisch und entzündungshemmend wirken. Gleiches gilt für die Wirkung von Nlcotinamid und eines Kondensationsprodukt aus Azelainsäure und Glycin. Beide Inhaltsstoffe wirken sebumreduzierend und dienen der Bekämpfung von Mitessern.

Insgesamt können so die angegebenen Inhaltsstoffe hinsichtlich ihrer Konzentrationen derart aufeinander abgestimmt bzw. einzeln in ihrer Konzentration verringert werden, dass bei gleichbleibender Wirksamkeit das Risiko einer Unverträglichkeit oder einer Hautreizung ausgeschlossen oder zumindest verringert ist. Zusätzlich können durch die Herabsetzung der Konzentration einiger Inhaltsstoffe die Kosten bei der Herstellung der Produkte gesenkt werden. So kann beispielsweise eine Verringerung der Konzentration des Phytospingosins, welches teuer und zudem nur bedingt wasserlöslich ist, die Herstellungskosten minimieren, was sich wiederum positiv auf den Verbraucher auswirkt. In der angegebenen Zusammensetzung ist Phytosphingosin mit einem Anteil von weniger als 1 Gew.-% enthalten.

In Untersuchungen konnte gezeigt werden, dass die Zusammensetzung, insbesondere im Einsatz zur Behandlung unreiner Haut bei Jugendlichen einen positiven Effekt hat. Nach einer regelmäßigen Anwendung konnte eine deutliche Verbesserung der Hautstruktur festgesteilt und auch die neuerliche Entstehung von Hautunreinheiten verringert werden.

Salicylsäure oder nach INCI-Deklaration Salicylic Acid zeigt für die Behandlung von Hautunreinheiten und im Speziellen von Akne einen guten keratolytischen und komedolytlschen Effekt. Zum einen werden bereits entstandene Hautunreinheiten wie Pickel und Mitesser durch den Einsatz von Salicylsäure bekämpft, da sie das Bakterienwachstum hemmt, zum anderen werden abgestorbene Zellen aus der Hornschicht entfernt und entzündliche Stellen ausgetrocknet. Zusätzlich ist Salicylsäure aufgrund der zellerneuernden Wirkung ebenfalls gut geeignet zur Behandlung reiferer Haut. Gleiches gilt insbesondere auch für anorganische Salze der Salicylsäure.

Nicotinamid oder nach INCI-Deklaration Niacinamide wird in der dermatologischen und kosmetisch orientierten Forschung im Hinblick auf Hautunreinheiten und Akne eingesetzt. Nicotinamid reguliert die Sebumproduktion, bekämpft Mitesser und Pickel und hat eine entzündungshemmende Wirkung. Nicotinamid ist auch als Vitamin-B3 bekannt.

Phytosphingosine gehören zur Familie der Lipide, die in der Epidermis vorkommen. Sie wirken ähnlich wie die Salicylsäure keratolytisch und entzündungshemmend. Zusätzlich versorgen Phytosphingosine die Haut mit Feuchtigkeit und mildern Hautrötungen im Zusammenhang mit Rosacea, Akne und entzündlichen Hautbildern.

Ein Kondensationsprodukt aus Azelainsäure oder nach INCI-Deklaration Azelaic Acid und Glycin oder nach INCI-Deklaration Glyzine, wirkt, wie auch Niacinamide, sebumreduzierend und dient der Bekämpfung von Mitessern. Als Kondensationsprodukt ist hierbei beispielsweise Azeloglycin oder nach INCI-Deklaration Potassium Azeloyl Diglycinate zu nennen. Azeloglycin verbindet die Eigenschaften der Azelainsäure mit der feuchtigkeitsspendenden Wirkung von Glycin und stelgert den Feuchtigkeitsgehalt und die Elastizität der Haut. Azeloglycin besänftigt die Haut, mildert Rötungen und hilft bei Unreinheiten. Zusätzlich verringert Azeloglycin die Talgproduktion und wirkt Mitessern entgegen.

Von Vorteil für die entzündungshemmende und sebumregulierende Wirksamkeit des Nicotinamids oder nach INCI-Deklaration Niacinamides ist es insbesondere, wenn die Zusammensetzung 1,0 - 3,0 Gew.-% hiervon umfasst.

Für die entzündungshemmende und keratolytische Wirkung und zur Verringerung des Risikos des Auftretens von Unverträglichkeiten hat es sich als vorteilhaft erwiesen, wenn die Zusammensetzung 0,1 - 0.3 Gew.-% Phytosphingosin umfasst.

Weiter vorteilhaft für die Verringerung der Talgproduktion und der Verhinderung der Entstehung von Mitessern hat sich in der Zusammensetzung ein Anteil von 2,0 - 5,0 Gew.-% des Kondensationsproduktes aus Azelainsäure oder nach INCI-Deklaration Azelaic Acid und Glycin oder nach INCI-Deklaration Glyzine oder seiner Salze erwiesen.

Vorzugsweise ist das anorganische Salz der Salicylsäure ein Alkalisalz. Beispielsweise kann Natriumsalicylat eingesetzt werden, welches als Keratolytikum wirkt und sich somit zur topischen Anwendung von Hautunreinheiten eignet.

Vorteilhafterweise ist von der Zusammensetzung weiter Panthenol mit einem Anteil von 0,5 - 2,0 Gew.-% umfasst. Panthenol, auch Dexpanthenol genannt, erhöht das Feuchthaltevermögen der Haut und verbessert deren Elastizität. Weiterhin unterstützt Panthenol die Neubildung der Hautzellen und trägt so zur Regeneration der Haut bel. Darüber hinaus wirkt Panthenol juckreizlindernd, entzündungshemmend und fördert die Wundheilung.

Zur Beruhigung gereizter Haut ist es weiter von Vorteil, wenn die Zusammensetzung Aloe Vera oder nach INCI-Deklaration Aloe Barbadensis in einem Anteil von 5 - 10 Gew.-% umfasst. Aloe Vera ist ein wirkungsvoller Träger und verbessert die Aufnahme anderer Inhaltsstoffe. Aloe Vera wird traditionell zur äußeren Behandlung von Akne, Ekzemen oder schlecht heilenden Wunden verwendet.

Zweckmäßigerweise umfasst der Rest der Zusammensetzung Grundstoffe und/oder Hilfsstoffe und/oder Duftstoffe. Als Grund- bzw. Hilfsstoffe sind zum Beispiel Emulgatoren, Konsistenzgeber, Emollients, Polymere etc, eingesetzt.

Die eingesetzten Grund- und Hilfsstoffe können belspielsweise bei der Herstellung einer Zusammensetzung bestimmte Fertigungsschritte verbessern und/oder eine ausreichende Haltbarkeit einer Zusammensetzung gewährleisten. Auch Eigenschaften wie Farbe, Geruch, Verträglichkeit usw. können durch derartige Grund- bzw. Hilfsstoffe eingestellt werden.

In einer weiter vorteilhaften Ausgestaltung der Erfindung umfasst die Zusammensetzung zusätzlich 0,1 - 2,0 Gew.-% Andean-Quillaja Saponin Extrakt oder nach INCI-Deklaration Quillaja Saponaria Bark Extract. Das Andean-Quillaja Saponin Extrakt ist ein rein pflanzliches Extrakt aus dem chilenischen Seifenrindenbaum, welches sich als geeignet zur Behandlung von Hautunreinheiten und insbesondere Akne gezeigt hat. Andean-Quillaja Saponin Extrakt zeichnet sich durch eine außerordentliche Hautverträglichkeit auch bei vorgeschädigter Haut aus. Das Extrakt wirkt feuchtigkeitsspendend und reguliert den Sebumgehalt der Haut, ohne sie auszutrocknen.

Die vorbeschriebene Wirkung des Andean-Quillaja Saponin Extrakts ist vergleichbar mit der Wirkung des Kondensationsprodukts aus Azelainsäure und Glycin. Somit kann durch die Zugabe des Andean-Quillaja Saponin Extrakts insbesondere die Konzentration des Kondensationsprodukts verringert oder gänzlich auf diesen Inhaltsstoff verzichtet werden, ohne dass sich dies auf die gewünschte Wirksamkeit der Zusammensetzung auswirkt. Mit anderen Worten kann das Andean-Quillaja Saponin Extrakt bei gleichbleibender Wirkung der Zusammensetzung alternativ oder zusätzlich zum Kondensationsprodukte aus Azelainsäure und Glycin eingesetzt werden. Dies ist Insbesondere von Vorteil, da das Andean-Quillaja Saponine Extrakt als pflanzlicher Wirkstoff üblicherweise keine Hautreizungen und Unverträglichkeiten hervorruft.

Die vorbeschriebene Zusammensetzung wird insbesondere zur unterstützenden Pflege von unreiner Haut, insbesondere bei Jugendlichen, angewendet. Die Zusammensetzung kann insbesondere in Hautpflegeprodukten wie Cremes oder dergleichen eingesetzt werden und so der Vorbeugung und Bekämpfung von Hautunreinheiten und Entzündungen dienen.

Die zweitgenannte Aufgabe wird erfindungsgemäß gelöst durch eine kosmetische und/oder dermatologische Zubereltung zur unterstützenden Pflege von unreiner

Haut, insbesondere bei Jugendlichen, mit einer Zusammensetzung der vorbeschriebenen Art.

Eine derartige kosmetische und/oder dermatologische Zubereitung ermöglicht die Pflege von unreiner Haut durch die Vorbeugung und die Behandlung von Entzündungen. Hierbei können die für die Zusammensetzung genannten vorteilhaften Ausgestaltungen sinngemäß auf die kosmetische und/oder dermatologische Zubereitung übertragen werden.

In einer vorteilhaften Ausgestaltung ist die kosmetische und/oder dermatologische Zubereitung ein Reinigungsgel. Ein Reinigungsgel entfernt schonend Fett- und Schmutzrückstände von der Haut und verringert so die Gefahr von Ablagerungen In den Poren, die zu Hautunreinheiten und Entzündungen führen können.

Weiter bevorzugt ist die kosmetische und/oder dermatologische Zubereitung als ein Gesichtswasser ausgeführt. Ein Gesichtswasser kann beispielsweise nach der Reinigung mit einem Reinigungsgel auf die Haut aufgetragen werden, um den reinigenden Effekt zu verstärken und die Haut zu entspannen.

In einer weiter vorteilhaften Ausgestaltung der Erfindung ist die kosmetische und/oder dermatologische Zubereitung eine Hautcreme. Die Hautcreme kann zur Pflege der Haut optimalerweise nach der Reinigung, beispielsweise mittels eines Reinigungsgels, und der zusätzlichen Anwendung eines Gesichtswassers auf die Haut aufgetragen werden.

Die Herstellung der kosmetischen und/oder dermatologischen Zubereitung erfolgt je nach gewünschtem Produkt auf verschiedene Weise. Zur Erläuterung der Erfindung sind im Folgenden Herstellungsverfahren für Ausführungsbeispiele der Erfindung beschrieben. Die Ausführungsbeispiele betreffen dabei ein Reinigungsgel, ein Gesichtswasser sowie eine Hautcreme. Die Inhaltstoffe der jewelligen Zusammensetzungen sind hierbei sowohl nach INCI-Deklaration als auch mit Handelsnamen benannt.

### (a) Herstellung eines Reinigungsgels

Zur Herstellung eines Reinigungsgels werden in einer Tensid-Phase A 8 Gew.-% Sodium Coco-Sulfate (Sulfopon 1216 G; EVONIK Industries), 22 Gew.-% Wasser, 0.2 Gew.-% Phytospingosine, 1 Gew.-% Salicylic Acid, 8 Gew.-% Disodium Cocoamphodiacetate (Rewoteric AM 2 C NM; EVONIK Industries), 1 Gew.-% Sucrose Cocoate (Tegosoft LSE65K soft; EVONIK Industries), 4,5 Gew.-% Coco-Glucoside (Plantacare 818 UP; BASF GmbH), 2 Gew.-% Sodium Cocoyl Glutamate (Plantapon ACG 50; BASF GmbH) gemlscht.

In einer wässrigen Phase B werden 44, 8 Gew.-% Wasser, 0,8 Gew.-% Hydroxypropyl Methylcellulose (Tegocel HPM 4000; EVONIK Industries), 0,5 Gew.-% Polyquaternium-10 (Jellner QH 300, DAICEL FINECHEM), 1 Gew.-% Panthenol, 2 Ges.-% Sodium Lactate, 3 Gew.% Potassium Azeloyl Diglycinate und 1 Gew.% Niacinamide gemischt.

Schließlich wird die Tensid-Phase A unter Rühren in der wässrigen Phase B emulgiert und abschließend Duftstoffe mit 0,2 Gew.-% hinzugefügt.

### (b) Herstellung eines Gesichtswassers

Zur Herstellung eines Gesichtswassers werden in einer ersten alkoholischen Phase A Ethanol, 1, 5 Gew.-% Salicyllc Acid, 0,1 Gew.-% Phytospingosin und 0,04 Gew.-% Duftstoffe gemischt.

In einer zweiten wässrigen Phase B werden 2 Gew.-% Niacinamide, 3 Gew.-% Glycerol, 10 Gew.-% Hamamelis Virginiana Extract, 1 Gew.-% Panthenol, 1 Gew.-% Coco-Glucoside (Plantacare 818 UP; BASF GmbH), 1 Gew.-% Sodium Cocoyl Hydrolyzed Wheat Protein (Gluadin WK; BASF GmbH), 4 Gew.-% Sodium Lactate 50 %-Ig, 2 Gew.-% Potassium Azeloyl Diglycinate und 51, 36 Gew.-% Wasser gemischt.

Abschließend werden die Phasen A und Phase B unter Rühren zusammengeführt.

### (b) Herstellung einer Hautcreme

Zur Herstellung einer Hautcreme als O-W-Emulsion werden in einer ersten öligen Phase A 3 Gew.-% Glycerin Stearat (Tegin pflanzlich; EVONIK Industries), 5 Gew.-% Ethylhexyl Stearat (Tegosoft OS: EVONIK Industries), 5 Gew.-% Dicaprylyl Carbonate (Cetiol CC; BASF GmbH), 1,6 Gew.-% Cetearyl Alcohol (Tego Alkanol 6855; EVONIK Industries / Lanette O; BASF GmbH), 1 Gew.-% Salicylic acid und 0,2 Gew.-% Phytospingosin bei einer Temperatur von 60° - 70 °C gemischt.

In einer zwelten Phase B werden 0,05 Gew.-% Aloe Pulver, 2,5 Gew.-% Polypropylenglycol (PPG)-1- Polyethylenglycol (PEG)-9 Lauryl Glycol Ether (Eumulgin SG; BASF GmbH), 0,3 Gew.-% Carbomer (Tego Carbomer 134; EVONIK Industries). 1, 5 Gew.-% Panthenol, 0, 1 Gew.% Komplexbildner, 3 Gew.-% Glycerol 86.5%-ig. 3 Gew.-% Niacinamide und 5 Gew.-% Potassium Azeloyl Diglycinate in 68,55 Gew.-% Wasser ebenfalls bei einer Temperatur von 60° -70 °C gelöst.

Schließlich wird die ölige Phase A unter Rühren in der wässrigen Phase B emulgiert. Die Emulsion wird schließlich auf etwa 30°C abgekühlt und abschließend Duftstoffe mit 0,2 Gew.-% hinzugefügt.

## Patentansprüche

1. Zusammensetzung, umfassend 0,5 - 2,0 Gew.-% Salicylsäure oder nach INCI-Deklaration Salicylic Acid oder ihre anorganischen Salze, 0,5 - 5,0 Gew.-% Nicotinamid oder nach INCI-Deklaration Niacinamide, 0,05 - 0,5 Gew.-% Phytosphingosin oder nach INCI-Dekiaration Phytosphingosine, 0 - 5,0 Gew.-% eines Kondensationsproduktes aus Azelainsäure oder nach INCI-Deklaration Azelaic Acid und Glycin oder nach INCI-Deklaration Glyzine oder seine Salze, sowie einen Rest

2. Zusammensetzung nach Anspruch 1,
umfassend 1,0 - 3,0 Gew.-% Nicotinamid oder nach INCI-Deklaratlon Niacinamide.

3. Zusammensetzung nach Anspruch 1 oder 2,
umfassend 0,1 - 0,3 Gew.-% Phytosphingosin oder nach INCI-Deklaration Phytosphingosine.

4. Zusammensetzung nach einem der vorhergehenden Ansprüche, umfassend 2,0 - 5,0 Gew.-% des Kondensationsproduktes aus Azelainsäure oder nach INCI-Deklaration Azelaic Acid und Glycin oder nach INCI-Deklaration Glyzine oder seine Salze.

5. Zusammensetzung nach einem der vorhergehenden Ansprüche,
wobei das anorganische Salz der Salicylsäure ein Alkalisalz und/oder ein Erdaikalisalz ist.

6. Zusammensetzung nach einem der vorhergehenden Ansprüche,
wobei zusätzlich Panthenol in einem Anteil von 0,5-2,0 Gew.-% umfasst ist.

7. Zusammensetzung nach einem der vorhergehenden Ansprüche,
wobei zusätzlich Aloe Vera oder nach INCI-Deklaration Aloe Barbadensis in einem Anteil von 5-10 Gew.-% umfasst ist.

8. Zusammensetzung nach einem der vorhergehenden Ansprüche,
wobei der Rest Grundstoffe und/oder Duftstoffe und/oder Hilfsstoffe umfasst.

9. Zusammensetzung nach einem der vorhergehenden Ansprüche,
wobei zusätzlich 0,1 - 2,0 Gew.-% Andean-Quilaja Saponine Extrakt oder nach INCI-Deklaratlon Quillaja Saponarla Bark Extract umfasst ist.

10. Zusammensetzung nach einem der Ansprüche 1 bis 9 zur unterstützenden
Pflege von unreiner Haut, insbesondere bei Jugendlichen.

11. Kosmetische und/oder dermatologische Zubereitung zur unterstützenden Pflege von unreiner Haut, insbesondere bei Jugendlichen, mit einer Zusammensetzung nach einem der Ansprüche 1 bis 9.

12. Kosmetische und/oder dermatologische Zubereitung nach Anspruch 11, die eine Hautcreme ist.

13. Kosmetische und/oder dermatologische Zubereitung nach Anspruch 11, die ein Reinigungsgel ist.

14. Kosmetische und/oder dermatologische Zubereitung nach Anspruch 11, die ein Gesichtswasser ist.
